# EUROPEAN PATENT SPECIFICATION

(11) **EP 4 376 497 B1**
(45) Date of publication and mention of the grant of the patent: **04.11.2026**
(21) Application number: 24169186.4
(22) Date of filing: 12.06.2019
(51) Int. Cl.: H04W 12/37, H04W 12/50, H04W 12/63, H04W 52/00, A61B 5/07, A61B 5/00, A61B 5/117, A61N 1/372, A61N 1/378

(54) **MANAGING SECURITY OF AN IMPLANTED MEDICAL DEVICE**
VERWALTUNG DER SICHERHEIT EINES IMPLANTIERTEN MEDIZINISCHEN GERÄTS
GESTION DE LA SÉCURITÉ D'UN DISPOSITIF MÉDICAL IMPLANTÉ

(30) Priority: 12.06.2018 US 201862683677 P
(43) Date of publication of application: 29.05.2024
(62) Document number(s) of the earlier application(s) in accordance with Art. 76 EPC: 19736818.6 / 3 808 120
(73) Proprietor: Impulse Dynamics NV, Willemstad (CW)
(72) Inventor: PRUTCHI, David, Voorhees, NJ 08043 (US); MEYERS, Jason, Haddonfield, NJ 08033 (US)
(74) Representative: Cohausz & Florack

(56) References cited:
- WO-A1-2017/007642
- US-A1- 2013 110 008

## Description

### FIELD AND BACKGROUND OF THE INVENTION

The present invention, in some embodiments thereof, relates to a method of securing wireless communication and, more particularly, but not exclusively, to a method of security key transfer with an implanted medical device over a near field communication channel.

U.S. Published Patent Application no. 20070118188 appears to disclose "A method and system for enabling secure communications between an implantable medical device (IMD) and an external device (ED) over a telemetry channel. A telemetry interlock may be implemented which limits any communications between the ED and the IMD over the telemetry channel, where the telemetry interlock is released when the ED transmits an enable command to the IMD via a short-range communications channel requiring physical proximity to the IMD. As either an alternative or addition to the telemetry interlock, a data communications session between the IMD and ED over the telemetry channel may be allowed to occur only after the IMD and ED have been cryptographically authenticated to one other."

U.S. Published Patent Application no. 20140185805 appears to disclose "Methods and systems for securely exchanging cipher keys between an implantable device and an external device ... An example method includes: receiving an authorization request from the external device, wherein the authorization request is a request to receive a first cipher key of a cipher key transfer; receiving an indication that a magnet is detected relative to the implantable device, wherein the indication signifies a secure environment for communication between the implantable device and the external device; and after receiving the authorization request and the indication of a detected magnet, generating a first cipher key transmittal instruction, wherein the first cipher key transmittal instruction instructs the first cipher key to be transmitted to the external device by the implantable device."

U.S. Patent no. 9154002 appears to disclose, "A wireless power supply system that detects communications in the input power to the switching circuit. In this aspect of the invention, the wireless power supply includes a detector for generating a signal indicative of the current in the input to the switching circuitry, a band-pass filter for filtering the detected signal, an amplifier for amplifying the filtered signal, a filter for filtering the amplified signal and a comparator for converting the final signal into a stream of high and low signals that can be passed to a controller for processing as binary data stream. In a second aspect, the wireless power supply system includes a detector for generating a signal that varies in dependence on changes in the phase relationship between the current and the voltage in the primary-side tank circuit, a band-pass filter for filtering the signal, an amplifier for amplifying the filtered signal, a filter for filtering the amplified signal and a comparator for converting the final signal into a stream of high and low signals that can be passed to a controller for processing as binary data stream."

U.S. Patent no. 5455466 appears to disclose, "A system for inductively coupling power and data to a portable electronic device. A portable device, such as a personal digital assistant, is powered or recharged via an inductive coupling between the device and a support unit, thereby eliminating the need for cabling or other connections therebetween. The same inductive coupling is also used to transfer data signals between the device and a second electronic device, for example, a conventional desktop computer. The support unit includes a primary winding of a transformer, a power amplifier and a modulator. The portable device includes a secondary winding connected in parallel with the input of a rectifier, the output of which is connected to a battery charging circuit, and to a modem, which is further connected to the device microprocessor. Placement of the device on the support unit effects the inductive coupling when the primary and secondary windings are in proximity to one another."

Document WO2017/007642 A1 discloses an implantable medical device detecting its location in a safety zone or high-risk zone and adapting its communication protocol accordingly. a receiver may detect that the user is in a location where the risk of eavesdropping is low and may transition to a higher-bandwidth or lower-power communication protocol without traffic-analysis protection. Particular locations may be designated as high-risk zones. In response to a determination that the user is in a high-risk zone, a mimicry protocol is initiated.

Document US 2013/110008 A1 relates to authentication and privacy for communicating with implanted medical devices.

### SUMMARY OF THE INVENTION

The invention is defined by the independent claims. Further embodiments are defined by the dependent claims.

The following passages relate to first to thirtieth aspects which do not define the scope of the present invention but merely pertain to the present disclosure.

A first aspect of the present disclosure relates to a method of secure communication between an implanted device and an external device comprising:
transferring energy over a transcutaneous energy transfer (TET) link to an implanted device by the external device;
modulating a verification key onto to said TET link by said external device; and encrypting communication over a radio channel using said verification key.

A second aspect of the present disclosure, as a variant of the first aspect, relates to a method, wherein the radio channel includes a range at least twice as large as the TET link.

A third aspect of the present disclosure, as a variant of any one of the first to second aspect, relates to a method, wherein the TET link uses at least twice as much power for a transmission as the radio channel.

A fourth aspect of the present disclosure, as a variant of any one of the first to third aspect, relates to a method, wherein the TET link requires at least twice as time for a transmission as the radio channel.

A fifth aspect of the present disclosure, as a variant of any one of the first to fourth aspect, relates to a method, wherein said transferring energy further includes: inducing a current in an implanted device by the external device.

A sixth aspect of the present disclosure, as a variant of any one of the first to fifth aspect, relates to a method further comprising charging a battery of said implanted device with said transferred energy.

A seventh aspect of the present disclosure, as a variant of any one of the first to sixth aspect, relates to a method, wherein said verification key is a public key and wherein said encrypting includes transmitting a message from said implanted device over said radio channel using asymmetric encryption and said public key.

An eighth aspect of the present disclosure, as a variant of the seventh aspect, relates to a method , wherein said message includes a session key, the method further comprising encrypting a command to said implanted device with said session key.

A ninth aspect of the present disclosure, as a variant of the seventh aspect, relates to a method, wherein said message includes a session key, the method further comprising encrypting data sent from said implanted device with said session key.

A tenth aspect of the present disclosure, as a variant of any one of the first to ninth aspect, relates to a method further comprising securing a command sent to said implanted device according to a high level security protocol; and securing data sent from said implanted device to an external device according to a low level security protocol.

An eleventh aspect of the present disclosure, as a variant of the tenth aspect, relates to a method, wherein a command from said external device to said implanted device to change a treatment parameter is assigned said high security level.

A twelfth aspect of the present disclosure, as a variant of the eleventh aspect, relates to a method, wherein said command is temporarily assigned said low security level in response to a condition of a user of the implanted device.

A thirteenth aspect of the present disclosure, as a variant of the twelfth aspect, relates to a method, wherein said condition includes a cardiac infarction.

A fourteenth aspect of the present disclosure, as a variant of any one of the tenth to thirteenth aspect, relates to a method, wherein said high level security protocol requires modulation of a renewed verification key within 15 minutes before accepting said command.

A fifteenth aspect of the present disclosure, as a variant of any one of the first to fourteenth aspect, relates to a method further comprising: verifying a message sent over said unsecured radio channel by sending a verification message from said external device to the implanted device over said TET link.

A sixteenth aspect of the present disclosure relates to an implanted device for secure communication comprising: a transcutaneous energy transfer (TET) receiver configured receiving power from and external device and supplying said power to the implanted device; a data receiving circuit connected to said TET receiver configured to receive a public key from said TET receiver; an encryption module functionally connected to said data receiving circuit for receiving said public key from said data receiving circuit and configured for encrypting a message with asymmetric encryption based on said public key to produce an encrypted message, and a transceiver functionally connected to receive said encrypted message from said transceiver and send said encrypted message to said external device over a two way radio channel.

A seventeenth aspect of the present disclosure, as a variant of the sixteenth aspect, relates to a device, wherein the implanted device does not include a modulator capable of modulating an outgoing message onto said TET channel.

An eighteenth aspect of the present disclosure, as a variant of any one of the sixteenth to seventeenth aspect, relates to a device, wherein the implanted device does not include an asymmetric decryption circuit capable of generating said public key and a private key and decrypting an asymmetric encrypted message encrypted with said public key.

A nineteenth aspect of the present disclosure, as a variant of any one of the sixteenth to eighteenth aspect, relates to a device further comprising a rechargeable power supply for said implanted device, said power supply functionally attached to said TET receiver for recharging from said power supplied by said external device.

A twentieth aspect of the present disclosure, as a variant of any one of the sixteenth to nineteenth aspect, relates to a device, wherein said external device includes a TET generator configured to transmit energy to the implanted device and an asymmetric decryption circuit capable of generating said public key and a private key and decrypting an asymmetric encrypted message encrypted with said public key and a modulator functionally connected to said decryption circuit for receiving said public key and said modulator functionally connected to said TET generator for modulating said public key onto a TET signal and transferring said key to the implanted device.

A twenty-first aspect of the present disclosure, as a variant of the twentieth aspect, relates to a device, wherein said external device does not include a receiver capable of receiving a message over said TET channel.

A twenty-second aspect of the present disclosure relates to a method of managing security of an implanted device comprising: detecting a current location; adjusting a security protocol according to said current location.

A twenty-third aspect of the present disclosure, as a variant of the twenty-second aspect, relates to a method, wherein said location is a high risk location and said adjusting includes increasing security limitations.

A twenty-fourth aspect of the present disclosure, as a variant of any one of the twenty-second to twenty-third aspect, relates to a method, wherein said location is a low risk location and said adjusting includes decreasing security limitations.

A twenty-fifth aspect of the present disclosure relates to a system to perform the method of any one of the twenty-second to twenty-fourth aspect.

A twenty-sixth aspect of the present disclosure relates to a method of managing security of an implanted device comprising: detecting a current condition of a user of the device; adjusting a security protocol according to said current condition.

A twenty-seventh aspect of the present disclosure, as a variant of the twenty-sixth aspect, relates to a method, wherein said condition is stable and said adjusting includes increasing security limitations.

A twenty-eighth aspect of the present disclosure, as a variant of any one of the twenty-sixth to twenty-seventh aspect, relates to a method, wherein said condition includes an acutely dangerous condition and adjusting includes decreasing security limitations.

A twenty-ninth aspect of the present disclosure relates to a system to perform the method of any one of the twenty-sixth to twenty-eighth aspect.

A thirtieth aspect of the present disclosure relates to a system for secure communication between an implanted device and an external device comprising:
an implanted device including an inductive energy receiving circuit; a data receiving circuit capable of demodulating a signal from said energy receiving circuit; a transceiver for data communication over a radio channel; and a processor configured for: encrypting a message with an asymmetric protocol; generating a symmetric encryption key; encrypting data using a said symmetric encryption key to produce an encrypted signal decrypting a received data signal using said symmetric encryption key; and a near field external device including, an induction generating circuit configured for inducting a current on said inducting energy receiving circuit.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

As will be appreciated by one skilled in the art, aspects of the present disclosure may be embodied as a system, method or computer program product. Accordingly, aspects of the present disclosure may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, some embodiments of the present disclosure may take the form of a computer program product embodied in one or more computer readable medium(s) having computer readable program code embodied thereon. Implementation of the method and/or system of some embodiments of the disclosure can involve performing and/or completing selected tasks manually, automatically, or a combination thereof. Moreover, according to actual instrumentation and equipment of some embodiments of methods, systems, and/or computer program products of the present disclosure, several selected tasks could be implemented by hardware, by software or by firmware and/or by a combination thereof, e.g., using an operating system.

For example, hardware for performing selected tasks according to some embodiments of the present disclosure could be implemented as an integrated circuit (for example a chip). As software, selected tasks according to some embodiments of the present disclosure could be implemented as a plurality of software instructions being executed by a computer using any suitable operating system. In an exemplary embodiment, one or more tasks according to some exemplary embodiments of method and/or system as described herein are performed by a data processor, such as a computing platform for executing a plurality of instructions. Optionally, the data processor includes a volatile memory for storing instructions and/or data and/or a nonvolatile storage, for example, a magnetic hard-disk and/or removable media, for storing instructions and/or data. Optionally, a network connection is provided as well. A display and/or a user input device such as a keyboard or mouse are optionally provided as well.

Any combination of one or more computer readable medium(s) may be utilized for some embodiments. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium and/or data used thereby may be transmitted using any appropriate medium, including but not limited to wireless, wireline, optical fiber cable, RF, etc., or any suitable combination of the foregoing.

Computer program code for carrying out operations for some embodiments of the present disclosure may be written in any combination of one or more programming languages, including an object oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Some embodiments of the present disclosure may be described below with reference to flowchart illustrations and/or block diagrams of methods, apparatus (systems) and computer program products. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

### BRIEF DESCRIPTION OF THE SEVERAL VIEWS OF THE DRAWING(S)

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIG. 1 is a schematic block diagram of a system and method of communication with an implanted device in accordance with an embodiment of the current invention;
FIG. 2 is a schematic block diagram of a system and method of communicating in accordance with an embodiment of the current invention;
FIG. 3 is a block diagram of system for communicating in accordance with an embodiment of the current invention;
FIG. 4 is a circuit diagram of system for communicating in accordance with an embodiment of the current invention;
FIG. 5A and 5B are schematic diagrams illustrating signal flow and/or security protocols in accordance with an embodiment of the current invention;
FIG. 6 is a block diagram of an implanted medical device in accordance with an embodiment of the current invention; and FIG. 7 is a flowchart illustration of a method of securing a communication in accordance with an embodiment of the current invention; and
FIGs. 8A-C are schematic diagrams illustrating security modes in accordance with an embodiment of the current invention.

### DESCRIPTION OF SPECIFIC EMBODIMENTS OF THE INVENTION

The present invention, in some embodiments thereof, relates to a method of securing wireless communication and, more particularly, but not exclusively, to a method of security key transfer with an implanted medical device over a near field communication channel.

### Overview

An aspect of some embodiments of the current invention relates to a security protocol for leveraging a one way intrusion resistant channel to secure communication between an implanted medical device and an external device on a less secure channel. In some embodiments, the secure channel will include a transcutaneous energy transfer (TET) link (for example including an inductive coupling). For example the TET link may be secured to prevent intrusion (for example preventing an unauthorized party from transmitting fraudulent communications on the channel). Optionally, a security key will be transferred over the intrusion resistant channel. For example, the security key may be used to secure information transferred over a separate channel, for example a one and/or two way radio channel. Optionally, there will be different levels of security on the key transfer and/or the key itself (how strong is the key) that are required for different communications.

In some embodiments, different levels of security will apply for different types of messages, different locations, different times and/or under different conditions (for example when a dangerous medical condition is detected the device may allow short term reprogramming that it would not allow under normal conditions). Optionally, some communications over the unprotected channel will only be implemented after confirmation over the intrusion resistant channel.

In some embodiments, the IMD may require authentication before receiving an encryption key. Optionally the authentication may be data based and/or non-data based. For example, the authentication may require functions that would be difficult to replicate by an intruder device. For example, a powerful transmitter may be required to be located very close to IMD. For example, transfer of the security may be initiated only when the IMD receives a sufficient quantity of energy and/or sufficient power and/or for a sufficient time over a TET channel. Alternatively or additionally, the security key transfer may only be initiated according to instructions passed over a separate channel. For example, initiation of a key transfer may require a command and/or a key passed to the IMD through a different channel. For example, the timing of the key transfer may be limited to a time transmitted over the two way data channel. Alternatively or additionally, the key transfer by require the ED to specify a session sequence from a communication over another channel. Optionally, an IMD may include a location detection device (e.g. a GPS) and/or may only accept a security key in a predetermined location.

In some embodiments, an asymmetric public key is optionally sent from the ED to the IMD over the intrusion resistant channel. The asymmetric key is optionally used for encrypted communication over another non-safe second channel. Asymmetric encryption may be used to send a session key from the IMD to the ED and/or the session key may be used for further communication. For example, the security key may be used to encrypt data and/or commands being sent over a MedRadio [MICS] channel. Optionally, the intrusion resistant channel may include a very short range channel (for example based on inductive coupling). In some embodiments the IMD may not be capable of generating a pair of keys for asymmetric communication keys. In some embodiments the IMD may not be capable of using a private key to decrypt a message encrypted with an asymmetric public key. For example, the processor of the IMD may be too weak for asymmetric decryption and/or may lack software instructions for asymmetric decryption.

In some cases, for example, when the IMD detects an emergency medical situation, certain communications may be allowed with an abbreviated security protocol. Optionally, some functions may be controlled only so long as an inductive device is in communication with the IMD. In some embodiments, certain functions may require security clearance including a security key passed over the protected channel.

In some embodiments, an IMD may have various security states and/or have functions that require different security levels. For example, to change life affecting settings of the IMD may require high security clearance, for example by use of a fresh security key and/or a key received over a protected channel. Alternatively or additionally, the ED receiving data from the IMD may be possible using an older security key. Alternatively or additionally, the IMD may have an emergency mode which allows changing of important (and/or life affecting) parameters with a lower security for a limited time. Alternatively or additionally, the security requirements for certain actions may be adjustable by a user having a sufficient security level. Optionally, an ED may have security protection such as a password and/or a bio-metric identifier to prevent unauthorized access. Alternatively or additionally, some aspects of the ED may require less or no security (for example charging a battery of the IMD) while other functions (for example viewing data) may need require medium level security (for example supplying a password) while other functions (for example reprogramming the IMD) may require high level security (for example biometric identification and/or a strong password).

For example, the ED may include a secure channel (for example a TET link) and/or a non-secure channel (for example a radio channel). A secure channel optionally includes a characteristic which makes it difficult for a hidden device to receive and/or transmit a signal. For example, the secure channel may be intrusion resistant. For example, an intrusion resistant channel may include a very short range communication medium (for example inductive coupling). For example the range of the secure channel may be less than ½ and/or less than 1/5 and/or less than 1/10 and/or less than 1/20 of the range of the non-secure channel. In some embodiments, an intrusion resistant channel may require high levels of power to transfer a signal. For example, the IMD may require transfer of enough power to charge a battery of the IMD before accepting a signal over the secure channel. For example transferring a message over the secure channel may require more than twice the energy and more than 5 times and/or more than 10 times and/or more than 20 times the energy for transferring the signal over the non-secure channel. In some embodiments communication may require a large time. For example, the IMD may require long contact time before accepting communication over the secure channel. For example transferring a message over the secure channel require more than twice the time and more than 20 times and/or more than 100 times and/or more than 1000 times the time for transferring the signal over the non-secure channel.

In the some embodiments, the ED will include security features to prevent misuse of the device. For example, the features may be activated to prevent unauthorized use of the ED for reprograming of the IMD. For example, the ED may include a biometric identification system. Optionally, the ED is programmed to change certain parameters of the ED only after positive identification and/or approval of a local user and/or approval of a supervisor (e.g. a doctor and/or a control center). In some embodiments, actions of the ED and/or the IMD are logged and/or data is sent to a control center. For example the logs may be checked manually and/or automatically to detect unusual activity and/or potentially dangerous situations.

An aspect of some embodiments of the current invention relates to a system for protecting communication between an IMD and an ED using infiltration resistant Transcutaneous Energy Transfer (TET) link. For example, the TET link may be configured for one way energy transfer from the ED to a battery on the IMD and/or one way communication from the ED to the IMD. In some embodiments, the system includes a two way radio link between the IMD and the ED. Optionally, the ED includes a processor configured for asymmetric key generating and decryption and/or the IMD includes a processor configured for asymmetric encryption based on a public key. For example, the public key may be supplied to the IMD over the TET link. Optionally, the ED includes a processor configured for symmetric key generating and encryption/decryption. Alternatively or additionally a key may be supplied to the ED from an external source (for example a network and/or an electronic data storage device).

In some embodiments, the IMD lacks circuitry and/or software to modulate a data signal onto the TET channel. Optionally, the ED lacks circuitry or software to receive a data signal over the TET channel.

In some embodiments, an IMD may include a therapeutic device for example a pacemaker and/or an implantable defibrillator, a neurostimulator, a cochlear implant, a gastric stimulator, a pump (e.g. an insulin pump), a foot drop implant, and/or a cardiac contractility modulation CCM device. Alternatively an implanted device may include a sensor.

An aspect of some embodiments of the current invention relates to an implanted device that adjusts its security according to a condition of a subject. For example, for a subject in a healthy state, the device may employ high level security protocols to protect data and/or prevent tampering with device functions. Alternatively or additionally, the device may accept commands and/or transfer data with lower security protocols at particular times and/or under certain conditions. For example, security may be reduced when there is an emergency medical condition that may require emergency and/or lifesaving intervention. For example, the device may include a sensor that senses a medical state of a subject and/or a processor that interprets sensor data and/or controls security.

An aspect of some embodiments of the current invention relates to an implanted device that adjusts its security according to a location. For example, in certain predetermined locations the device may reduce security requirements (for example security limitations may be reduced in a low risk location, for example in the home of the subject and/or security limitations may be reduced in a location where therapeutic interventions are expected, for example an emergency room and/or in a doctor's office). Alternatively or additionally, in certain locations the device may increase its security for example, in a foreign country and/or near an embassy of a hostile country and/or in a high crime area. In some embodiments, the implanted device may include a location sensor (for example a GPS) and/or security may be adjusted according to the location. Alternatively or additionally, certain locations (for example a doctor's office and/or an emergency room) may have location indicator devices that are detected by the IMD.

### Embodiments

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

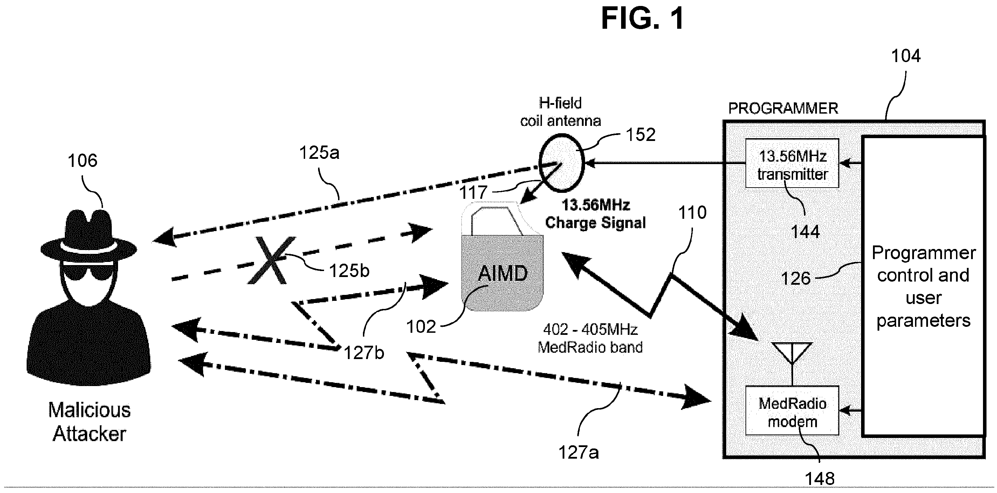

Referring now to the drawings, *Figure 1* illustrates a schematic view of a system and method of securing communication between an implanted medical device (IMD) **102** and an external device (ED)**104** from infiltration by an intruder **106.** In some embodiments, IMD **102** communicates with ED **104** over multiple wireless media. For example a one way intrusion resistant medium **117** may be used for communication from the ED **104** to the IMD **102.** For example, the ED **104** may include a transmitter **144** configured for one way transmission to the IMD which may include a receiver for receiving signals over the intrusion resistant channel. Optionally the intrusion resistant channel includes a TET link. In some embodiments the length of transmission on the intrusion resistant channel may be limited, for example to between 1 to 5 cm and/or between 5 to 15 cm and/or between 15 to 100 cm. For example, a non-secure medium **110** may be used for two way communication. For example, the ED **104** and/or IMD **102** may include a radio transceiver **148.** For example, receiver **148** may be configured for two way communication over a MedRadio band (e.g. between 402 to 405 MHz). In some embodiments the length of these transmissions may be limited, for example to between 1 to 3 meters and/or between 3 to 30m and/or between 30 to 100 meters and/or between 100 to 1000 meters.

In some embodiments, signals from the ED **104** to the IMD **102** may include control commands and/or performance parameters **126** for the IMD **102.** Parameters **126** are optionally stored in the ED **104.** For example, in a computer accessible memory and/or received from a remote source and/or from a local programmer and/or generated by a local and/or remote processor. For example, operating parameters of the IMD **102** may be adjusted according to data received from the ED **104.** In some embodiments the IMD may include a read/write computer readable member for storing alternative parameter values and/or a clock (for example a real time clock). For example, the IMD may receive a temporary set of parameters from the ED. The IMD may then store a current set of parameters in the computer readable memory and/or the IMD may use the temporary parameters for a predetermined period and reinstate the previous parameters after the period.

In some embodiments, the intrusion resistant medium **117** may be protected from infiltration **125b.** Nevertheless, in some embodiments, the intrusion resistant medium **117** may be vulnerable to interception **125a.** For example intrusion resistant medium **117** may include a TET link. The TET link may be used to charge a power supply (for example a battery) of the IMD. For example, the inductive coupling may be used for the power transfer. Additionally or alternatively, the ED **104** may transmit signals to the IMD **102** over channel **117.** For example, the ED may include a transmitter **144** to transmit signals over the TET link at carrier frequency between 12-14 MHz or more or less. Optionally the TET link between the ED **104** and the IMD **102** may be limited in range for example to less than 5 cm and/or less than 10 cm and/or less than 30 cm and/or less than 1 m.

In some embodiments, the currently-disclosed invention is configured to secure communication under the assumption that communications between the IMD **102** and external device **104** on the intrusion resistance medium **117** can be conceivably received **125a** by a malicious eavesdropper **106** (for example with sufficiently sensitive receivers). The malicious attacker **106** could conceivably transmit **127a, 127b** signals via MedRadio that can be received by the ED **104** and/or the IMD **102** at a distance. Nevertheless, the system is optionally configured to inhibit attacker **106** from controlling the IMD **102.** For example, the attacker may be prevented from transmitting a spoofed message **125b** to the IMD **102** over the intrusion resistant medium **117.** For example, to make such a transmission may require an unreasonably large H-field transmitter and/or approaching unreasonably close to the IMD **102.** For example the system prevents the attacker **106** from spoofing a programmer-to-IPG communication over the intrusion resistant channel **117** without being detected. For example, in some embodiments, a security protocol may amplify the limitations of the attacker **106.** For example, a security protocol may require a predetermined signal power and/or energy transfer before accepting an encryption key over the intrusion resistant channel **117.** For example, it may be difficult for a malicious attacker **106** to acquire that much power and/or transfer that much power to the IMD **102** when his device is hidden (for example requiring only a small power source and/or preventing connection to a fixed power cable).

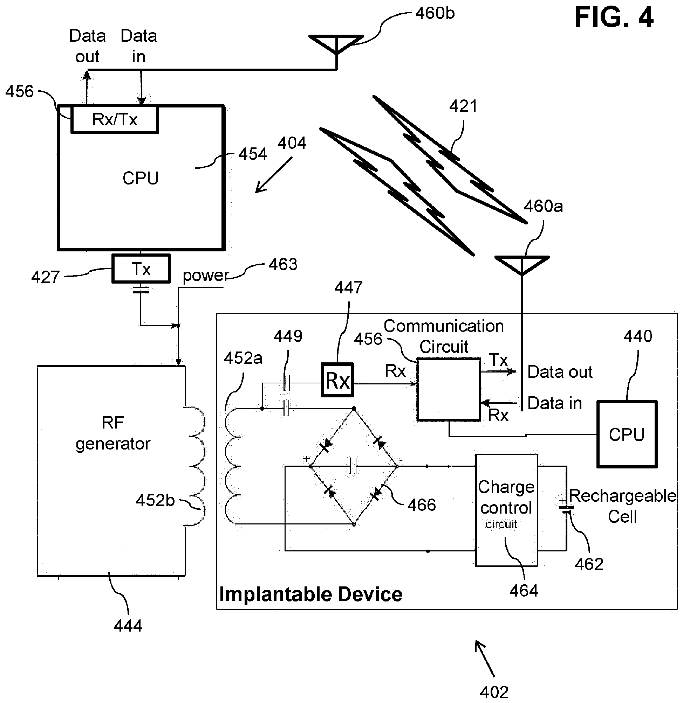

In some embodiments, the coupling on the intrusion resistant channel decays very rapidly with distance between the transmitter (e.g. a coil **152** in the ED) and the receiver (e.g. internal coil for example as illustrated by coil **452a** of *Fig.* 4) used to receive the transcutaneously-transmitted energy. For example, the recharge distance **104** between the external charger coil and the implant may be restricted to just a few centimeters (e.g. <5cm). As such, a malicious attacker **106** attempting to transmit data over the recharge channel to an AIMD over a larger distance would require a very large and powerful transmitter and antenna. The need to operate a large transmitter in the vicinity of the IMD may inhibit a surreptitious attack.

In some embodiments, transferring a key and/or verification for security communications at some predetermined level will be limited by time and/or location. For example, an IMD **102** may include a location determination circuit (for example a GPS receiver). The IMD **102** optionally only accepts passwords in a known protected location (for example in the house of the user and/or in one or more known locations for example a hospital and/or an office of a trusted practitioner). For example, this may prevent an attacker **106** from inviting a user of the IMD **102** to a location where attacker has a hidden transmitter with enough power to intrude into the system and/or spoof communications to the IMD **102** over channel **117.** In some embodiments, the IMD **102** may accept a key at some predetermined security level at specific times. For example, this may prevent an attacker **106** from sneaking up on a user of IMD **104** at an unexpected moment (for example while he is sleeping). In some embodiments, acceptance of a key at a certain security level by the IMD **102** will depend on a physiological state of the user of the IMD **102.** For example, the IMD **102** may not accept a key for changing a long term treatment parameter of the device while the user is asleep. For example, when the IMD **102** detects an emergency condition (for example a cardiac infarction, the IMD **102** may allow emergency short term changes in functioning parameters with abbreviated security protocol.

In some embodiments, performance of certain function by the IMD **102** may be dependent on the link to the ED **104.** Optionally, functions that are highly security sensitive and/or require high power may be performed only under certain circumstance including for example while the IMD **102** is receiving power from the ED **104** over the TET link. For example, communication of a session key using asymmetric encryption may be performed only under certain conditions including for example when the IMD **102** is receiving power on the TET link and/or when the battery power of the IMD **102** is at some minimum capacity.

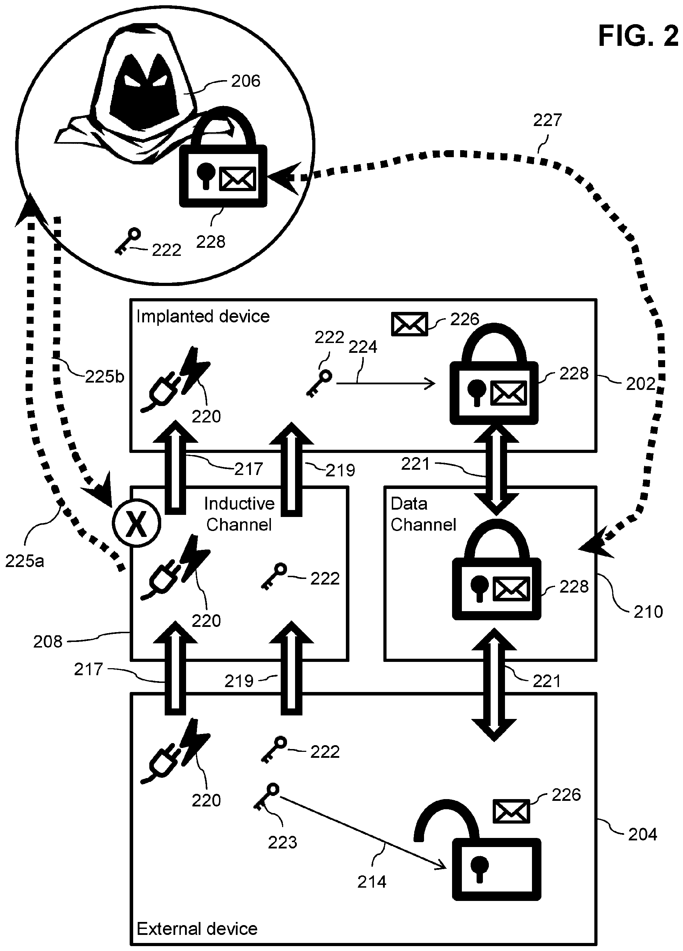

*FIG. 2* is a schematic block diagram of a system and method of communicating in accordance with an embodiment of the current invention. In some embodiments, a protected communication process will include a transfer (for example a cryptographic key-transfer **219)** between the IMD and a valid external device (e.g. ED **204).** This transfer may be made, for example, over the short-range channel **208** implemented over the TET (Transcutaneous energy transfer) link. Optionally the TET is used for to supply energy and/or to recharge **217** the IMD **202.** The transfer step is optionally designed to exploit the asymmetry between the valid ED **204** and the malicious intruder **206** to transmit data and/or energy **220** that can be received by the IMD **202.** For example, communication over the "long-range" channel is secured when the malicious attacker **206** is not able to "spoof" **225b** the transmission of data from the valid ED **204** to the IMD **202.** Optionally, the security system is designed such that interception **225a** of the data transmitted over the intrusion resistant link **208** shall not give a malicious attacker the means to compromise the security of the long-distance communication **221.**

In some embodiments, the availability of a known intrusion resistant short range communication link **208** is leveraged to securely encrypt communications over an unprotected channel **210** (for example, a MedRadio channel). For example, the intrusion resistant channel **208** is used to transfer an asymmetric public key **222.** For example the length of the asymmetric encryption public key **222** and/or private key **223** may range for example between 32 bits and 256 bits. Optionally, the system may use asymmetric (public key) cryptography **228** for the ED **204** and the IMD **102** to agree on a session encryption key. For example, the IMD may generate a session key and/or send the session key in a message **226** to the ED **204.** For example message **226** may encrypted **224** by IMD **202** using public key **222** and/or transmitted in an encrypted message **228,** but not transmitted unencrypted over either channel **208, 210.** Encryption with the asymmetric key inhibits the attacker from communicating **225b, 227** with the IPG even if the short range communication is intercepted **225a.** The ED **204** may receive the encrypted message **228** over the data channel **210** and/or decrypt **214** the data **226** (including for example the session key) using its private key **222.** Optionally private key **223** was never transmitted over any channel (for example over either channel **208** and/or **210).** For example, the private key **223** may be generated by and remains local to the ED **204.**

In some embodiments, two-way communication between the ED **204** and the IMD **206** is encrypted/decrypted with the session key. Optionally the session key may include for example between 32 to 256 key bits plus a number of auxiliary protocol bits. The session key may only be valid for a predefined short period of time (for example less than one minute and/or between 1 to 5 minutes and/or between 5 minutes to 30 minutes and/or between 30 minutes to 6 hours and/or between 6 hours to 48 hours and/or between 48 hours to a week and/or between a week and a month. Alternatively or additionally, for low security data, a session key may be used continuously between charging session of the IMD **202.** Renewing the session key periodically may prevent the use of brute force attacks or statistical methods to attack the MedRadio link. Optionally multiple session keys may be used simultaneously and/or after a certain period of time an old session key may be used for only low security data, but high security will require a renewed session key. In some embodiments, multiple session keys will be transferred in a charging session and/or switched between charging sessions. This strategy has the added benefit that the computational (and therefore power) demands placed on the IMD **202** to do the encryption are reasonably able to be implemented in a microcontroller. Optionally, of the more computationally intensive cryptographic processes are performed on the ED **204,** which will be built around a more powerful processor with less stringent energy limitations. This procedure allows communications between the IMD **202** and ED **204** to proceed over the MedRadio link while protecting the system from an attacker **206** remotely reprogramming the IMD **202.**

In some embodiments, even if an attacker **206** intercepts communication on one or both of the protected channel **208** and/or the two-way data channel **210,** he will be left with an asymmetric public encryption key **222** and/or encrypted data **228** but the attacker will be prevented from acquiring a decryption key (either the asymmetric decryption key **223** and/or a symmetric key that was transmitted in an encrypted form). Alternatively or additionally, communication **219** in the intrusion proof channel **208** may include verification of commands to the IMD **202.** For example, even if the attacker **206** is capable of spoofing a malicious command over the data channel **210** to command the IMD **202,** the IMD **202** will not carry out the command until it receives verification transmitted **219** by the legitimate ED **202** over the intrusion resistant channel **208.** Optionally verification may include a specific data for example repeating a parameter value which the IMD **202** is to implement. Optionally, when a suspicious incident occurs (for example a command is received but not verified), a warning message is transmitted by the IMD **202** to the ED **104** and/or to a security center.

In some embodiments, a system leverages an intrusion resistant channel **208** (for example a TET link) to secure communication between an IMD **202** and an ED **204** over another channel **210.** Optionally, the intrusion resistant channel may include an inductive coupling which may be used for intrusion resistant communication **219** and/or power transfer **217.** For example the power transfer **217** and/or communication **219** may be one-way (for example from the ED **204** to the IMD **202).** For example the power transfer may be at a rate ranging between 0.1 to 0.3 Watts and/or 0.3 to 1 Watt and/or 1 Watt to 5 Watts. Data rate of transmission over the TET channel may range for example between 50 to 200 bits/s and/or 200 to 1 Kbit/s and/or between 1 Kbit/s to 5 Kbit/s and/or between 5 Kbits/s to 20 Kbits/sec. A second communication channel **210** optionally supports two-way communication. For example the data transmission rate on the two-way communication channel **210** may range between 1kbit/s to **100** Kbit/s and/or between 100 Kbit/sec and/or from 100 Kbit/s to 1Mbit/s and/or between 1 to 5 Mbit/sec and/or between 5 to 25 Mbits/s. Optionally the carrier frequency of the communication channel may range between 402 to 405 and 433 to 435 MHz and/or between 2.4 GHz to 2.5 GHz. In some embodiments, the ED **204** may charge the IMD **202** with between 0.1 to 0.5 Watt hours and/or 0.5 to 1 Watt hour and/or between 0.5 to 10 Watt Hours of energy in a single session.

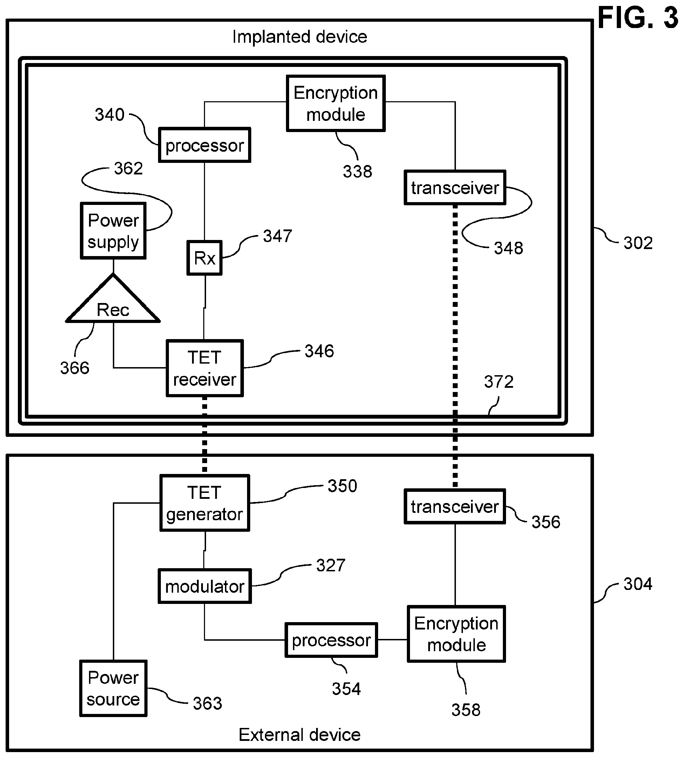

*FIG.* 3 is a block diagram of system for communicating in accordance with an embodiment of the current invention. In some embodiments an IMD **302** includes two communication modules. Optionally, a first communication module includes a signal receiver **347** connected to a TET receiver **346** for one way communication over TET channel (for example an inductive coupling). Optionally, a second communication module includes a transceiver **348** for two way communication, for example on a radio (e.g. radio wave and/or microwave) channel. For example, transceiver **348** may communicate with a transceiver **356** on the ED **304.** Optionally the TET receiver is also connected to a power supply **362** (for example including a rechargeable battery), for example via a rectifying circuit **366.** Optionally, IMD **302** is in communication with an ED **304.** For example the ED **304** may include a TET generator **350.** Optionally, TET generator **350** is connected to a power source **363.** Optionally, energy produced power source **363** is transferred by TET generator **350** to TET receiver **346** and/or rectified by rectifier circuit **366** and/or supplied to recharge power supply **362.** In some embodiments, ED **304** includes a modulator **327** connected to TET generator **350.** For example, a signal from modulator **327** may be transmitted via TET generator **350** over the TET coupling to TET receiver **346.** Optionally, the signal is picked up by signal receiver **347.**

In some embodiments, TET **350** is positioned outside a body of a patient at close range to TET receiver **346** which may be positioned inside the patient. For example, the distance between TET generator **350** and TET receiver **346** may range between 0 to 1 cm and/or between 1 to 2 cm and/or between 2 to 5 cm and/or between 5 to 8 cm and/or between 8 to 12 cm and/or between 12 to 20 cm.

In some embodiments, TET generator **350** and receiver **346** may include inductive coils. Optionally, receiver **347** may include a band pass filter and/or a modem and/or an amplifier and/or an analogue to digital converter and/or a universal asynchronous transmitter and receiver (UART). For example, rectifier circuit **366** may include a bridge rectifier circuit.

In some embodiments, IMD **302** may be encased in a biocompatible and/or water resistant casing **372.**

In some embodiments, an IMD **302** may include a processor **340.** Processor **340** optionally includes an encryption module **338.** Optionally encryption/decryption/key generation could be in either hardware, software, or a combination (i.e. software with hardware acceleration). For example module **338** may include programs stored in a computer readable memory to support transmitting asymmetrically encrypted data using a public key. Alternatively or additionally, encryption module **338** may include dedicated circuitry and/or a dedicated processor. Processor **340** and/or module **338 are** optionally, capable of symmetric encoding and/or decoding of data. For example, **processor 340** and/or module **338** may be capable of supporting encrypted communication at rate of between 0.1 to 0.5 Mbps and/or between 0.5 to 5 Mbps and/or between 5 to 20 Mbps and/or between 20 to 100 Mbps and/or generating encryption keys. Optionally, processor **340** and/or module **338** may be capable of communicating and processing messages encrypted with for example between 32 to 256 key bits plus a number or auxiliary protocol bits. Optionally the IMD **202** includes a recharge circuit capable of recharging the power storage in a time ranging between 10 minutest to 30 minutes and/or 30 minutes to 1 hour and/or between 1 to 4 hours. Processor **340** optionally is connected to and/or receives signals (which may optionally be digitized) from receiver **347.**

Processor **340** and/or module **338** optionally are connected to and/or send and/or receive signals through receiver **348.**

In some embodiments, the ED **304** may include a processor **354** and/or an encryption module **358** capable of decoding asymmetrically encoded communication for example encrypted with between 32 to 256 key bits and/or generating asymmetric encryption keys. Alternatively or additionally, the ED **304** may be capable of protected communication of passwords and/or asymmetrically encoded data with an external processor. For example communication may be over a protected medium (e.g. a hard wired link and/or by means of a data storage device (for example a USB drive). Optionally the power source **363** receives power from an external network for example an electrical power grid. Alternatively or additionally, the power source **363** may include a local power supply, for example a rechargeable cell with a capacity ranging 0.1 to **1.0** Watt hours and/or between 1.0 to 3 Watt hours and/or between 3 to 10 Watt hours and/or a single use cell with a capacity ranging 0.5 to 1.0 Watt hours and/or between 1.0 to 3 Watt hours and/or between 3 to 10 Watt hours and/or between 10 to 30 Watt hours.

*FIG. 4* is a circuit diagram of system for communicating in accordance with an embodiment of the current invention. In some embodiments, coil **452b** of an ED **404** receives power from a power source **463** and/or a one-way outgoing signal from an RF generator **444.** The power is optionally transferred over a TET link to the IMD **402.** For example, the TET link may transfer power and/or data inductively to an inductance coil **452a** of an IMD **402.** Optionally, coil **452a** is connected via a rectifying circuit **466** (for example a full bridge rectifier as depicted in *Fig.* 4) to a charge control circuit **464** and/or a rechargeable power source **462.** Alternatively or additionally, coil **452a** is connected to a signal receiver circuit **447** which demodulates a one-way incoming signal. For example, circuit **447** may be connected to coil **452a** via a tuned capacitor and/or band pass filter **449.** Data from circuit **447** is sent over a one-way link to a communication module **456.** Module **456 is** optionally configured for sending asymmetric encryption of outgoing signals over a radio transceiver **460a.** For example, outgoing asymmetrically encoding signals may be encoded using a public key received from receiver **447** and/or from the TET inductance channel from an ED. Module **456** is optionally configured for symmetric decryption and/or encryption of signals over a two way radio channel **421.** Optionally, transceiver **460a** includes a dedicated antenna. Alternatively or additionally, transceiver **460a** uses coil **452a** as a radio antenna. Communication is optionally controlled by a controller **440.** Optionally communication circuit **456** and/or controller **440** includes a memory for storing an asymmetric key received over the TET link.

In some embodiments, an ED **404** includes a two way transceiver **460b** for communicating of radio channel **421** with IMD **402.** Optionally, ED **404** includes a processor **454.** For example, processor **454** may be configured for asymmetric and/or symmetric encoding and/or decoding and/or for generating of keys for symmetric and/or asymmetric encryption/decryption. For example, processor **454** is connected to a module **427** for one-way encoding of an asymmetric public key and transfer of the public key to RF generator and/or over the TET link to IMD **402.** In some embodiments, at some times when asymmetric communication is not being used an asymmetric encoding module **456** and/or processor **440** may be shut down. For example, an asymmetric encoding module may be shut down when IMD **502** is not receiving power over the TET link. For example shutting down an asymmetric encryption module may save power.

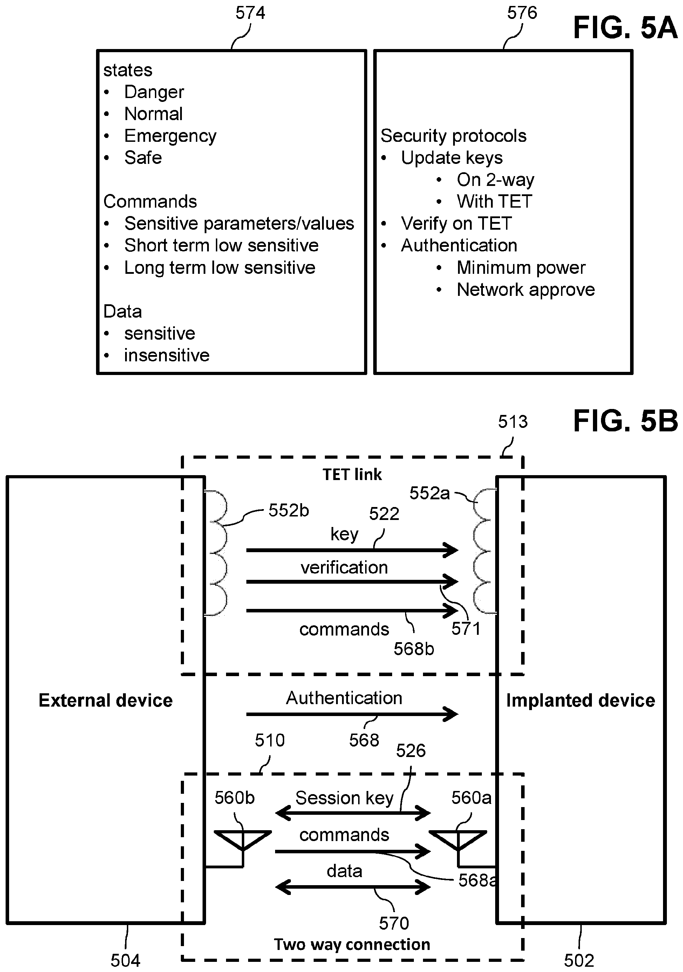

*FIG. 5A* is a schematic diagram illustrating signal flow and/or security protocols in accordance with an embodiment of the current invention. Various contents of communications and/or security states **574** may be protected by various security protocols **576** for example in accordance with a sensitivity and/or urgency of the communication.

In some embodiments, an IMD may have various security states. For example, the IMD may recognize a state in which there is an increased risk of malicious attack. For example, there may be an increased risk of malicious attack when the device (and/or the person to which the device is implanted) is in an unsecured location, for example where a malicious attacker may be able to position malicious hardware. For example, there may be an increased risk of malicious attack when the person in whom the device is implanted is asleep and/or at night when the user may be unaware of malicious hardware being positioned next to his body. Optionally, the IMD **568** may include positioning indicator (for example a GPS receiver and or a list of stored locations that are safe and/or unsafe) and/or a sensor to determine a state of the user (e.g. asleep, awake by a pulse sensor and/or a blood pressure sensor). For example, in a state with increased risk of attack, a device may not allow certain sensitive communications (for example a command that would shut down a lifesaving system and/or change a parameter of functioning of the IMD **502** that could endanger the user of the device and/or a permanent parameter changes). Alternatively or additionally, in a state where there is increased risk the IMD **502** may require increased security protocols and/or verification over a normal mode.

In some embodiments, an IMD **502** may have a normal mode. For example, in the normal mode, certain changes and/or communications may be allowed and/or other changes may be prohibited. Alternatively or additionally, certain security protocols may be in place to protect the data and/or commands from malicious attack.

In some embodiments an IMD **502** may have an emergency mode. For example, when an IMD **502** detects a symptom of a dangerous health condition (for example a myocardial infarction and/or an ischemia), the IMD **502** may enter an emergency mode. For example, in the emergency mode, the IMD **502** may take action to protect the user (for example to increase blood flow and/or stabilize cardiac activity). Alternatively or additionally, in the emergency mode, the IMD **502** may lower security and/or allow emergency and/or medical personnel to make short term changes in the functioning of the IMD **502.** Optionally, the IMD **502** may have a memory (read only and/or read write) that stores certain actions that are allowed in one or more emergency situations with reduced security. Optionally the IMD **102** may have a computer readable memory (for example a RW and/or RO memory) that stores the restore and/or default and/or current parameter values that can be restored after the temporary parameters expire and/or the emergency situation changes. Optionally, the IMD **102** may include a real time clock. For example the clock may be used to determine when a parameter value has expired and/or should be changed. In some embodiments, an IMD **502** may have a safe mode. For example, when the IMD **502** detects that it is in a safe location (for example a location of a recognized hospital and/or a home of the user). In the safe mode, the IMD **502** may have a lowered security allowing programming and/or data transfers that would not be allowed in the normal mode and/or with reduced security measure than in the normal mode. Optionally, the security protocols will remain unchanged, but certain functions may be assigned to different security levels according to a secondary condition. For example a secondary condition may include a location and/or a condition of the subject and/or a time.

In some embodiments, different commands and/or actions may require different levels of security. For example, a command to change a setting of the IMD **502** that may in a short term cause significant harm and/or danger to the user may require the highest level of security. For example, a long term change is a setting of the IMD **502** that could cause danger and/or harm to the user may require a high level of security. For example, a short term change in a setting of the IMD **502** and/or a change that is unlikely to cause significant harm or danger to a user may require a medium level of security. For example, communication of health and/or sensitive data may require a medium level of security. For example, communication of non-sensitive data (for example a battery level) may require low level of security.

In some embodiments, a different level of security may require a different security protocol. For example, a message at the highest security may require individual verification over the TET channel **513.** Alternatively or additionally, a message at the highest security may be allowed on a two way channel **510** when the security key is fresh (for example when the security key was fixed based on a communication over the TET channel within the last minute and/or within the last 10 minutes and/or within the last half hour and/or within the last six hours). Optionally, a message at a high level of security may be accepted based only on the security of the two way channel **510** and/or with an older security key than the highest security level, for example when the security key was fixed based on a communication over the TET channel within the within the last 10 minutes and/or within the last half hour and/or within the last six hours and/or within the last day and/or if the device has been in a safe location since the last security key refresh. Optionally, for medium and/or low level security an older key may be acceptable and/or even a non-secured communication link may be used.

In some embodiments, verification and/or key transfer on the TET channel may be secured by authentication. Optionally, authentication of TET communications may be required for high level security actions. For example, authentication may include requiring the TET channel to transfer a large amount of power and/or energy and/or to transfer power over a long time (something that may be particularly difficult for a malicious intruder). Alternatively or additionally, authentication may require use of a code or another verification of the identity of the ED **504.** Alternatively or additionally, authentication may include security verification over the two-way channel before accepting a security key over the TET. In some embodiments, the verification **571** may include the ED **504** sending a parameter value to the IMD **504** over the TET **513** link. In some embodiments, verification will include repeating a parameter value sent over the two way channel **510.** Alternatively or additionally, a command may be given over the two way channel **510** to change a parameter value and the new value may be given over the TET link **513.** Alternatively or additionally, a value may be given over the two-way channel **510** and a message defining which parameter to change may be sent over the TET link **513.** In some embodiment, an authentication may include a requirement of an operator identification. For example, an ED **504** may include a bio-metric device and/or an input device for identifying an operator. In some embodiments, an IMD **502** will allow a temporary change of state and/or therapeutic parameter prior to verification. For example, when verification is received in time, the new state may be preserved. Optionally, when verification is not received in a predetermined time, the IMD **502** may revert back to a previous state and/or parameter.

*FIG. 5B* is a schematic diagram illustrating signal flow and/or security protocols in accordance with an embodiment of the current invention. In some embodiments, a one-way communication channel over a short range TET link **513** is used for transferring **522** an encryption key for encryption of a message. The message may include, for example a session key **526** and/or a command **568a** and/or data **570** transmitted between an ED **504** and an IMD **502** over a two way communication channel **510.** Alternatively or additionally, the one-way communication TET link **513** may be used for verification **571** of a message sent from the ED **504** to the IMD **502** and/or for sending a command **568b** from the ED **504** to the IMD **502.** In some embodiment, communication over a TET is further protected by a authentication **568** protocol.

In some embodiments, a TET link **513** may be used for verification of a message over the two way channel **510.** For example, when ED **504** gives a highly sensitive command (for example a command to change a treatment parameter of the IMD **502)** the IMD **502** may require verification over the TET link **513.** For example, verification **571** may include a simple statement verifying that the ED **504** sent a command **568a** over the two way channel **510.** Alternatively or additionally, the verification message may include a password and/or a time stamp and/or a packet ID number that identifies the message from the two way link **510.**

In some embodiments, a session on the two way link **510** may have multiple security keys that change from time to time and/or according to instructions passed over the TET link **513** and/or according to instruction passed over an encrypted conversation in the two way channel **510** and/or according to stored data shared between the IMD **502** and the ED **504.** Switching of session keys from time to time may make it harder to break the encryption of the two way channel **510** by statistical means.

In some embodiments, the IMD **502** may periodically send a list of settings and/or treatment parameters to the ED **504.** For example the data may be checked periodically to make sure that no settings were inadvertently and/or maliciously mis-set.

In some embodiments, a limited range of changes in treatment settings of the IMD **502** may be permitted with a relatively low level of security while other changes may require higher security. For example, the IMD may include a read only and/or a read write memory with stored ranges of settings that are allowed with relatively low security. Alternatively or additionally, relatively small changes in parameters may be allowed with lower security than a larger change.

In some embodiments the TET link **513** may include an inductive channel. For example a signal and/or energy may be sent from an inductor (for example a coil **552b)** of the ED **504** to an inductor (for example a coil **552a)** of the IMD **502.** In some embodiments, the two-way channel **510** may include a radio channel. For example radio signals may be sent back and forth between a transceiver **560a** of the IMD **504** and a transceiver **560b** of the ED **504.** Optionally, transceiver **560a** may include a dedicated antenna. Alternatively or additionally, transceiver **560a** may use coil **562a** as an antenna.

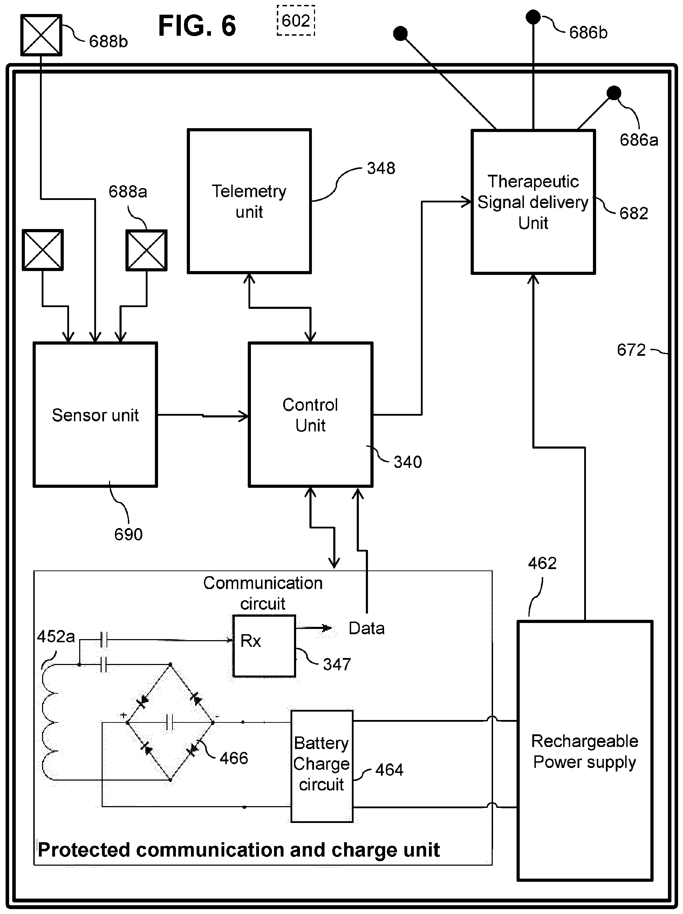

*FIG.* 6 is a block diagram of an IMD **602** in accordance with an embodiment of the current invention. In some embodiments, an IMD **602** includes a therapeutic unit **682** and/or a sensor unit **690.** For example, a therapeutic unit may include actuators **686a 686b** that apply therapies to tissue. For example, the sensor unit **690** may include sensors **688a 688b** which sense a condition of a user of the device.

In some embodiments an IMD **602** may be encased in a protective cover **672** (for example cover **672** may be water proof, biocompatible, protect the user from the internal parts of the IMD and/or protect the user from electric shock and/or to protect internal parts of the IMD **602** from body fluids and/or to protect the IMD from physical damage for example knocks). Optionally one or more sensors (for example sensor **688a)** are inside cover **672.** For example, sensor **688a** may sense a magnetic field. Alternatively or additionally, a sensor that extends outside of cover **672** (for example sensor **688b).** For example, sensor **688b** may include an electrode, pressure transducer, a thermocouple and/or a flow meter.

In some embodiments, one or more actuators (for example actuator **686a)** are inside cover **672.** For example, an actuator **688a** may produce a magnetic field. Alternatively or additionally, an actuator that extends outside of cover **672** (for example actuator **686b).** For example, actuator **686b** may include an electrode, an ultrasound transducer and/or a heating element. In some embodiments a single element may serve both as a sensor and an actuator. For example, an electrode may be used to collect information about electrical signal inside the user and/or also apply an electrical signal. For example, IMD may include a pacemaker and/or an implantable cardiac defibrillator (ICD) and/or a cardiac contractility modulation (CCM) device. For example, the device may apply pacing signals and/or non excitory signals at various periods of the cardiac cycle.

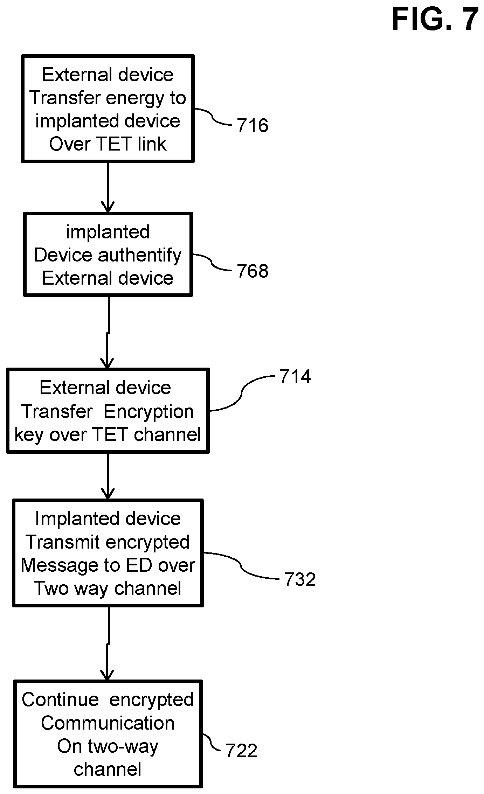

*FIG. 7* is a flowchart illustration of a method of securing a communication in accordance with an embodiment of the current invention. In some embodiments, an infiltration resistant TET channel will be used protect an IMD from being controlled by a malicious device and/or to protect communication between an IMD and an ED. For example, the infiltration protected TET channel will be used to transfer a public key to the IMD that will be used to prevent an attacker from infiltrating communication over a less protected channel (for example by encrypting communication over the less protected channel). Optionally, the TET channel may be configured for one way communication and/or the less protected channel may be configured for higher speed communication and/or two way communications.

In some embodiments, an external device will transfer energy **716** to an implanted device. For example the energy transfer may include power for charging a battery of the IMD. Optionally the energy transfer link may be over a channel that decays quickly over distance. Optionally, in order to spoof signals over the energy transfer link a malicious device may require high power and/or a position very close to the IMD. For example the link may lose more than 25% of its power over 10 cm and/or more than 50% of its power over 50 cm and/or more than 90% of its power over two meters. For example, the energy transfer may be over a TET link. For example, the TET link may include an inductive coupling.

In some embodiments, the IMD may authenticate **768** that the ED is legitimate device. For example, authentication may include requiring the ED to transfer a certain quantity of energy and/or power and/or to transmit power over a minimal time span. Alternatively or additionally, further authentication methods may be used.

In some embodiments, the ED may use the TET link to transfer **714** an encryption key to the IMD. For example, the ED may transfer a public key of asymmetric encryption to the IMD. Optionally the IMD will encrypt a message with the encryption key supplied by the ED. For example, the message may be sent from the IMD to the ED over a two-way communication medium. In some embodiments, the IMD will encrypt a session key using the encryption key supplied from the ED and/or transfer **732** the encrypted session key over the two-way communication medium to the ED. Further communication **722** will optionally proceed over the two-way using symmetric encryption based on the session key.

In some embodiments, security of continued communication on the two-way medium will be boosted using the TET link. For example, the ED may periodically send a new encryption key over the TET link and/or the new key will be used to refresh the old encryption keys, for example by encrypting and transferring a new session key. Alternatively or additionally, the TET link may be used to verify communications received from the ED. Alternatively or additionally, only a partial message may be sent over each medium. For example, the TET link may be used to transfer certain data such that the messages on either medium are not enough to understand the full message and/or carry out the instructions being communicated. For example, a message sent over the two-way link may lack a necessary parameter value that is sent over the TET link.

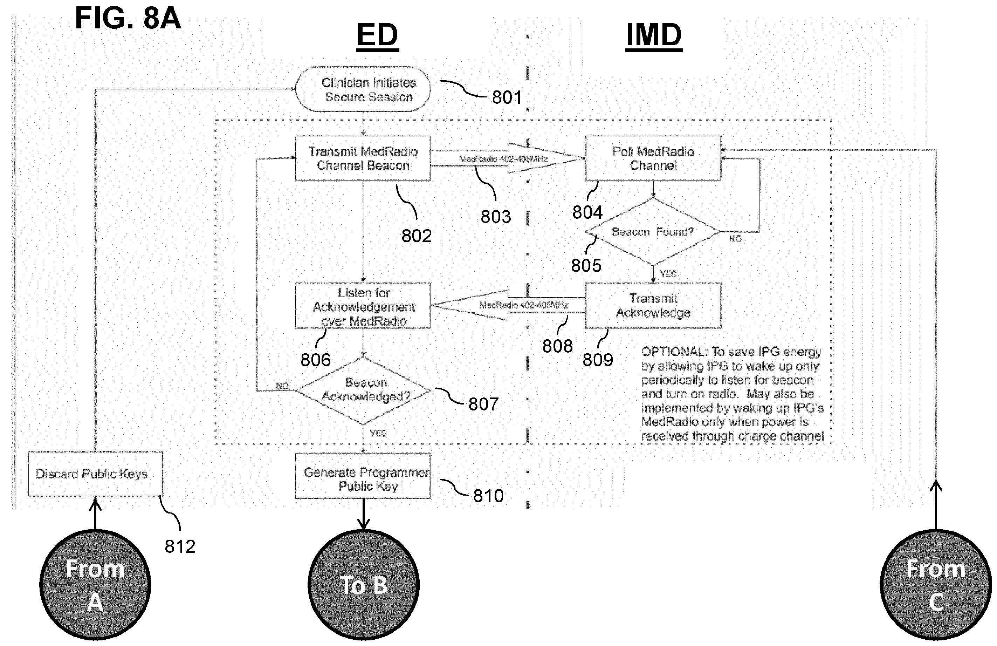

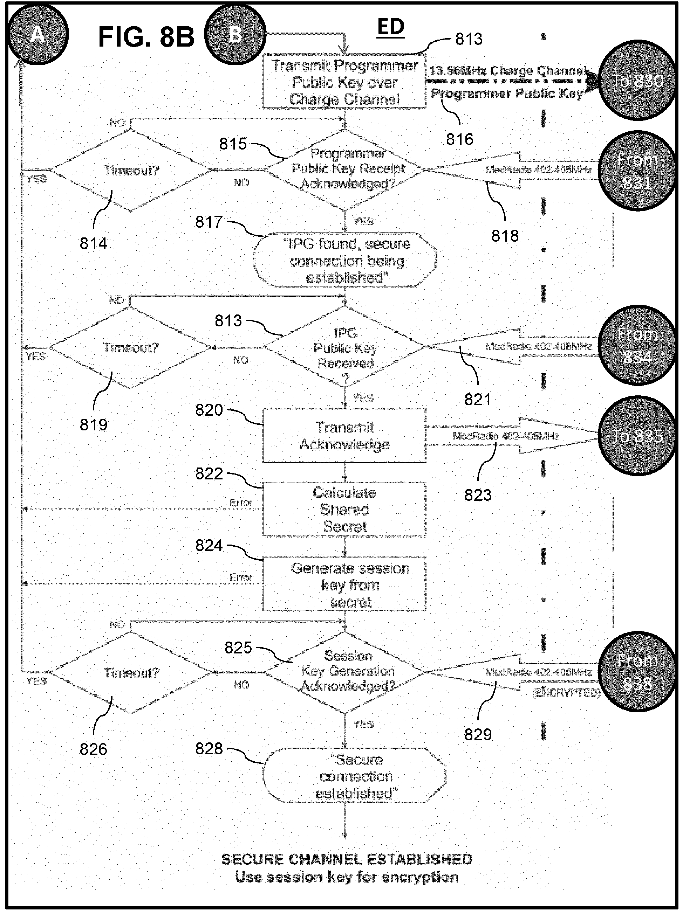

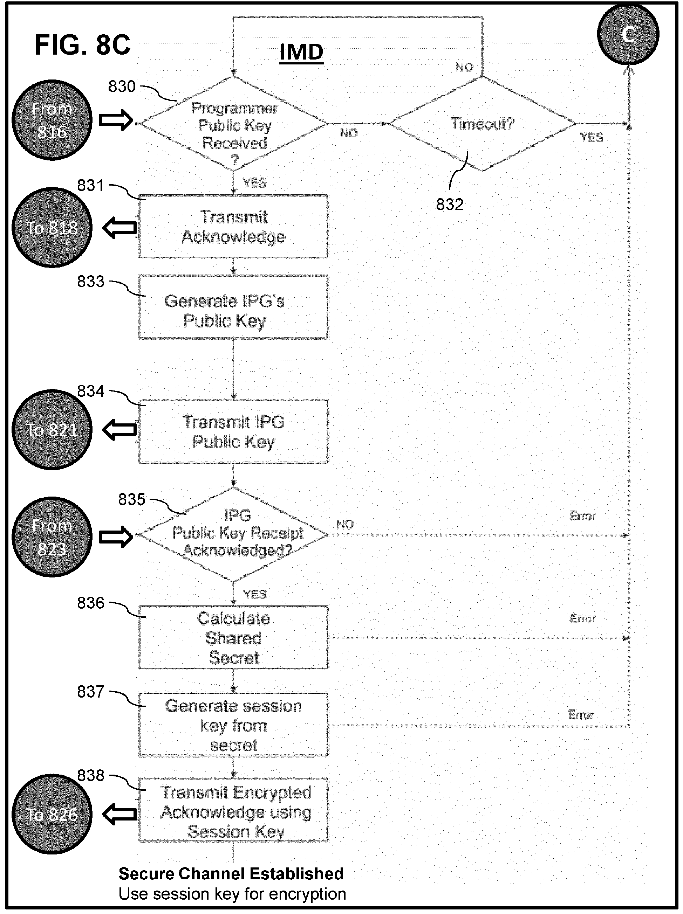

*FIGs. 8A-8C* illustrate an example of communication between an ED and an IMD in accordance with an embodiment of the current invention.

In some embodiments, a clinician initiates **801** a secure session. For example, the clinician moves the ED to a position alongside a subject near a location where the IMD is implanted. For the ED may be held within 10 cm of the IMD. A communication link may open automatically as a result of the proximity of the ED to the IMG. Alternatively or additionally, the clinician may activate the ED and/or the clinician may initiate charging the IMD over a TET link. Alternatively or additionally, the IMD may remain active polling **504** a communication channel **803** (without an external initiation).

In some embodiments, the session begins with the ED transmitting **802** a beacon signal **803** to the IMD. Optionally the signal may be a MedRadio signal (e.g. a 402-405 MHz signal). Optionally, the IMD is periodically polling **804** for the beacon. Alternatively or additionally, the ED may activate the communication of the IMD. For example the IMD may include a reed switch which is activated by a magnet in the ED and/or over the TET link. Optionally, after transmitting **802,** the beacon, the ED listens **806** for an acknowledgement.

In some embodiments, when the IMD is activated and/or has received **805** the beacon signal **803** the IMD sends **809** an acknowledgement signal **808** to the ED. For example, the acknowledgement may be over the MedRadio channel and/or another medium.

In some embodiments, when the ED receives **807** the beacon acknowledgement **808,** a public key is generated **810** and sent **813** to the IMD. For example the key is sent **513** over a secure channel **816.** Optionally, the secure channel **816** includes a medium on which it would be difficult for an intruder to transmit a counterfeit message. For example, example the secure channel **816** may require very short range between the transmitter and the IMD and/or may require high power levels and/or may require long connection time. For example, the secure channel **816** may include a TET channel, for example a 13.56 MHz charge channel. Optionally, when the IMD receives **830** the public key, it transmits **831** an acknowledgement **818** to the ED. Optionally upon receiving **815** the acknowledgement **818,** the ED displays **817** a message to the clinician, for example "IPG found secure connection being established." Optionally, if an acknowledgement **818** is not received **815** within a timeout **814** period, then the ED discards **812** current public keys and waits for initiation **801** of a new session by the clinician. If the public key is not in not received **830** in a timeout period **832** the IMD optionally returns to the polling **804** state.

In some embodiments, after transmitting **831** acknowledgement of receipt of the public key, the IMD generates **833** a public key and/or transmits **834** the public key **821** to the ED. For example, key **821** may be transferred over the MedRadio channel. The IMD optionally waits **835** for an acknowledgement **823** of receipt of the public key **821.** For example, the ED may transmit **820** acknowledgement **823** of receipt of the public key over the MedRadio channel. If there is an error, the system optionally returns to the waiting state, e.g. waits for the clinician to initiate **801** a new session. Optionally, if a key **821** is not received **813** within a timeout **819** period, the ED discards **812** current public keys and waits for initiation **801** of a new session from the clinician.

In some embodiments, after exchange of public keys, the IMD and/or the ED generate **822, 836** a shared secret. Optionally the shared secret is used to generate **824, 837** a session key. The session key is optionally used to encrypt further communication. For example, the IMD may transmit **838** an encrypted acknowledgement **829** of generating **837** the session key. If there is an error **825,** for example the session key of the ED fails to decrypt the acknowledgement **829** of the IMD and/or the acknowledgement **829** is not received within a timeout **826** period, then the public keys may be discarded **812** and/or the system may return to wait for new instructions to initiate **801** a secure session from the clinician. Once the secure session is established, the ED optionally displays **828** a success message to the clinician and/or communication continues using the session key. Alternatively or additionally, the ED may send an acknowledgement of successful generation of the session key to the IMD.

It is expected that during the life of a patent maturing from this application many relevant communication media and/or protocols will be developed and the scope of the terms radio channel and encryption is intended to include all such new technologies *a priori.*

As used herein the term "about" refers to ± 10%.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range. When two ranges are connected with an and/or connector then the ranges may be separate and/or continuous. For example if a parameter is said to range between 1 to 3 and/or between 3 to 5 and/or 5 to 7 then the ranges 1 to 5 and 5 to 10 and 1 to 10 are also included.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

## Claims

1. A method of managing security of an implanted medical device implanted in a patient, comprising:
- detecting a current location of said implanted medical device;
- adjusting, at said implanted medical device, a security protocol of said implanted medical device according to said detected current location,
**characterized by**
- accepting, at said implanted medical device at said detected current location, reception of a security key via an intrusion resistant first communication channel; and
- using said received security key for securing information transferred from said implanted medical device over a second communication channel, said second communication channel being different from said first communication channel.

2. The method of claim 1, wherein
- said location is a high risk location and said adjusting includes increasing security limitations,
or
- said location is a low risk location and said adjusting includes decreasing security limitations.

3. The method according to claim 3, wherein at least one of the following applies:
- said low risk location is a hospital, emergency room, doctor's office or patient's home; and
- said location is detected by said implanted medical device detecting a location indicator device.

4. The method according to any of claims 1-5, comprising:
- recognizing, by the implanted medical device, a state in which there is an increased risk of malicious attack; and
- requiring, by the implanted medical device, increased security protocols and/or verification over a normal mode of the device.

5. The method according to claim 4, wherein at least one of the following applies:
- said recognizing comprises recognizing based on a sleep state of said patient and/or based on a time being night, and
- in a state with increased risk of attack certain sensitive communications are not allowed.

6. The method according to any of claims 1-5, comprising:
- detecting, by the device, a symptom of a dangerous health condition; and
- entering an emergency mode by said device.

7. The method according to claim 6, wherein, in the emergency mode, the device may lower security and/or allow emergency and/or medical personnel to make short term changes in the functioning of the device and, optionally, after the emergency mode is over, restoring one or more device parameters from a computer readable memory of the device.

8. The method according to claim 7, wherein said restoring is based on time measured by a real time clock in the device.

9. The method according to any of the preceding claims, wherein, in a normal mode, said device allows certain changes and/or communications and prohibits other changes.

10. The method according to any of the preceding claims, wherein said device allows a short term change with a lower level of security relative to a change with may cause significant harm and/or danger to the patient.

11. The method according to any of the preceding claims, wherein a different level of security requires a different security protocol including one or more of a fresh security key, authentication on an external device and authentication by a transcutaneous energy transfer, TET communication, for higher security levels.

12. The method according to any of the preceding claims, wherein at least one of the following applies:
- said intrusion resistant first communication channel is a transcutaneous energy transfer, TET, link,
wherein, in particular, at least one of the following applies:
- said transcutaneous energy transfer, TET, link includes an inductive coupling,
- said transcutaneous energy transfer, TET, link uses an external device for transferring energy to said implanted medical device,
- said transcutaneous energy transfer, TET, link forms a very short-range channel,
- said transcutaneous energy transfer, TET, link loses more than 25% of its power over 10 cm and/or loses more than 50% of its power over 50 cm and/or loses more than 90% of its power over two meters.

13. The method according to any of the preceding claims, wherein at least one of the following applies:
- said detected location is in close proximity to an external device for transferring energy to said implanted medical device over a link forming said intrusion resistant first communication channel,
- said detected location is within 10 cm of an external device for transferring energy to said implanted medical device over a link forming said intrusion resistant first communication channel.

14. The method according to any of the preceding claims, wherein at least one of the following applies:
- an external device for transferring energy to said implanted medical device over a link forming said intrusion resistant first communication channel is brought near said detected location, and
- a communication link between said implanted medical device and said external device opens automatically as a result of a proximity of said implanted medical device to said external device.

15. An implantable device configured to carry out the method of any of claims 1-14 and configured for one or more of pacing the heart, defibrillating the heart and applying cardiac contractility modulation signals to the heart.

## Patentansprüche

1. Verfahren zur Verwaltung der Sicherheit eines in einen Patienten implantierten medizinischen Geräts, umfassend:
- Erfassen einer aktuellen Position des implantierten medizinischen Geräts;
- Anpassen eines Sicherheitsprotokolls des implantierten medizinischen Geräts in dem implantierten medizinischen Gerät entsprechend der erfassten aktuellen Position,
**gekennzeichnet durch**
- Akzeptieren, in dem implantierten medizinischen Gerät an der erfassten aktuellen Position, des Empfangens eines Sicherheitsschlüssels über einen gegen Eindringen resistenten ersten Kommunikationskanal; und
- Verwenden des empfangenen Sicherheitsschlüssels zum Sichern von Information, die von dem implantierten medizinischen Gerät über einen zweiten Kommunikationskanal übertragen wird, wobei sich der zweite Kommunikationskanal von dem ersten Kommunikationskanal unterscheidet.

2. Verfahren nach Anspruch 1, wobei
- die Position eine Position mit hohem Risiko ist und das Anpassen ein Erhöhen von Sicherheitsbeschränkungen umfasst,
oder
- die Position eine Position mit niedrigem Risiko ist und das Anpassen ein Verringern von Sicherheitsbeschränkungen umfasst.

3. Verfahren nach Anspruch 2, wobei mindestens eines des Folgenden zutrifft:
- die Position mit niedrigem Risiko ist ein Krankenhaus, eine Notaufnahme, eine Arztpraxis oder das Zuhause des Patienten; und
- die Position wird dadurch erfasst, dass das implantierte medizinische Gerät eine Positionsanzeigevorrichtung erfasst.

4. Verfahren nach einem der Ansprüche 1-5, umfassend:
- Erkennen, durch das implantierte medizinische Gerät, eines Zustands, in dem ein erhöhtes Risiko eines böswilligen Angriffs besteht; und
- Erfordern, durch das implantierte medizinische Gerät, erhöhter Sicherheitsprotokolle und/oder einer erhöhten Verifizierung gegenüber einem Normalmodus des Geräts.

5. Verfahren nach Anspruch 4, wobei mindestens eines des Folgenden zutrifft:
- das Erkennen umfasst ein Erkennen basierend auf einem Schlafzustand des Patienten und/oder basierend darauf, dass es Nacht ist; und
- in einem Zustand mit erhöhtem Angriffsrisiko sind bestimmte sensible Kommunikationen nicht zulässig.

6. Verfahren nach einem der Ansprüche 1-5, umfassend:
- Erfassen, durch das Gerät, eines Symptoms eines gefährlichen Gesundheitszustands; und
- Eintreten des Geräts in einen Notfallmodus.

7. Verfahren nach Anspruch 6, wobei das Gerät in dem Notfallmodus die Sicherheit herabsetzen und/oder Notfall-Personal und/oder medizinischem Personal kurzfristige Änderungen an der Funktionsweise des Geräts erlauben kann und, optional, nach Beendigung des Notfallmodus einen oder mehrere Geräteparameter aus einem computerlesbaren Speicher des Geräts wiederherstellt.

8. Verfahren nach Anspruch 7, wobei das Wiederherstellen auf einer durch eine Echtzeituhr in dem Gerät gemessenen Zeit basiert.

9. Verfahren nach einem der vorhergehenden Ansprüche, wobei das Gerät in einem Normalmodus bestimmte Änderungen und/oder Kommunikationen erlaubt und andere Änderungen untersagt.

10. Verfahren nach einem der vorhergehenden Ansprüche, wobei das Gerät eine kurzfristige Änderung mit einem niedrigeren Sicherheitsniveau im Vergleich zu einer Änderung erlaubt, die dem Patienten erheblichen Schaden zufügen und/oder eine erhebliche Gefahr für den Patienten verursachen kann.

11. Verfahren nach einem der vorhergehenden Ansprüche, wobei ein unterschiedliches Sicherheitsniveau ein unterschiedliches Sicherheitsprotokoll erfordert, das für höhere Sicherheitsniveaus eines oder mehrere von einem neuen Sicherheitsschlüssel, einer Authentifizierung an einer externen Vorrichtung und einer Authentifizierung durch eine Kommunikation zur transkutanen Energieübertragung, TET, umfasst.

12. Verfahren nach einem der vorhergehenden Ansprüche, wobei mindestens eines des Folgenden zutrifft:
- der gegen Eindringen resistente erste Kommunikationskanal ist eine Verbindung zur transkutanen Energieübertragung, TET,
wobei insbesondere mindestens eines des Folgenden zutrifft:
- die Verbindung zur transkutanen Energieübertragung, TET, umfasst eine induktive Kopplung,
- die Verbindung zur transkutanen Energieübertragung, TET, verwendet eine externe Vorrichtung zum Übertragen von Energie an das implantierte medizinische Gerät,
- die Verbindung zur transkutanen Energieübertragung, TET, bildet einen Kommunikationskanal mit sehr kurzer Reichweite,
- die Verbindung zur transkutanen Energieübertragung, TET, verliert über 10 cm mehr als 25 % ihrer Leistung und/oder verliert über 50 cm mehr als 50 % ihrer Leistung und/oder verliert über zwei Meter mehr als 90 % ihrer Leistung.

13. Verfahren nach einem der vorhergehenden Ansprüche, wobei mindestens eines des Folgenden zutrifft:
- die erfasste Position befindet sich in unmittelbarer Nähe zu einer externen Vorrichtung zum Übertragen von Energie an das implantierte medizinische Gerät über eine Verbindung, die den gegen Eindringen resistenten ersten Kommunikationskanal bildet,
- die erfasste Position befindet sich innerhalb von 10 cm von einer externen Vorrichtung zum Übertragen von Energie an das implantierte medizinische Gerät über eine Verbindung, die den gegen Eindringen resistenten ersten Kommunikationskanal bildet.

14. Verfahren nach einem der vorhergehenden Ansprüche, wobei mindestens eines des Folgenden zutrifft:
- eine externe Vorrichtung zum Übertragen von Energie an das implantierte medizinische Gerät über eine Verbindung, die den gegen Eindringen resistenten ersten Kommunikationskanal bildet, wird in die Nähe der erfassten Position gebracht; und
- eine Kommunikationsverbindung zwischen dem implantierten medizinischen Gerät und der externen Vorrichtung öffnet sich automatisch infolge einer Nähe des implantierten medizinischen Geräts zu der externen Vorrichtung.

15. Implantierbare Vorrichtung, die dazu konfiguriert ist, das Verfahren nach einem der Ansprüche 1-14 auszuführen, und die für eines oder mehrere von einer Stimulation des Herzens, einer Defibrillation des Herzens und einem Anlegen von Signalen zur Modulation der Herzkontraktilität an das Herz konfiguriert ist.

## Revendications

1. Procédé de gestion de la sécurité d'un dispositif médical implanté chez un patient, comprenant :
- détecter un emplacement actuel dudit dispositif médical implanté ;
- ajuster, au niveau dudit dispositif médical implanté, un protocole de sécurité dudit dispositif médical implanté en fonction dudit emplacement actuel détecté,
**caractérisé par**
- accepter, au niveau dudit dispositif médical implanté audit emplacement actuel détecté, la réception d'une clé de sécurité via un premier canal de communication résistant aux intrusions ; et
- l'utilisation de ladite clé de sécurité reçue pour sécuriser des informations transférées depuis ledit dispositif médical implanté via un deuxième canal de communication, ledit deuxième canal de communication étant différent dudit premier canal de communication.

2. Procédé selon la revendication 1, dans lequel
- ledit emplacement est un emplacement à haut risque et ledit ajustement comprend une augmentation des limitations de sécurité,
ou
- ledit emplacement est un emplacement à faible risque et ledit ajustement comprend une diminution des limitations de sécurité.

3. Procédé selon la revendication 2, dans lequel au moins l'un des éléments suivants s'applique :
- ledit emplacement à faible risque est un hôpital, un service d'urgence, un cabinet médical ou le domicile du patient ; et
- ledit emplacement est détecté par ledit dispositif médical implanté détectant un dispositif indicateur d'emplacement.

4. Procédé selon l'une quelconque des revendications 1 à 5, comprenant :
- reconnaître, par le dispositif médical implanté, un état dans lequel il existe un risque accru d'attaque malveillante ; et
- exiger, par le dispositif médical implanté, des protocoles de sécurité et/ou une vérification accrus par rapport à un mode normal du dispositif.

5. Procédé selon la revendication 4, dans lequel au moins l'un des éléments suivants s'applique :
- ladite reconnaissance comprend une reconnaissance basée sur un état de sommeil dudit patient et/ou sur le fait qu'il fait nuit ; et
- dans un état présentant un risque accru d'attaque, certaines communications sensibles ne sont pas autorisées.

6. Procédé selon l'une quelconque des revendications 1 à 5, comprenant :
- détecter, par le dispositif, un symptôme d'un état de santé dangereux ; et
- faire entrer ledit dispositif dans un mode d'urgence.

7. Procédé selon la revendication 6, dans lequel, dans le mode d'urgence, le dispositif peut abaisser le niveau de sécurité et/ou permettre au personnel d'urgence et/ou au personnel médical d'effectuer des modifications à court terme du fonctionnement du dispositif et, facultativement, après la fin du mode d'urgence, restaurer un ou plusieurs paramètres du dispositif à partir d'une mémoire lisible par ordinateur du dispositif.

8. Procédé selon la revendication 7, dans lequel ladite restauration est basée sur le temps mesuré par une horloge en temps réel dans le dispositif.

9. Procédé selon l'une quelconque des revendications précédentes, dans lequel, dans un mode normal, ledit dispositif autorise certaines modifications et/ou communications et interdit d'autres modifications.

10. Procédé selon l'une quelconque des revendications précédentes, dans lequel ledit dispositif autorise une modification à court terme avec un niveau de sécurité inférieur par rapport à une modification susceptible de causer un préjudice important et/ou un danger pour le patient.

11. Procédé selon l'une quelconque des revendications précédentes, dans lequel un niveau de sécurité différent exige un protocole de sécurité différent comprenant, pour des niveaux de sécurité plus élevés, un ou plusieurs parmi une nouvelle clé de sécurité, une authentification sur un dispositif externe et une authentification par une communication de transfert d'énergie transcutané, TET.

12. Procédé selon l'une quelconque des revendications précédentes, dans lequel au moins l'un des éléments suivants s'applique :
- ledit premier canal de communication résistant aux intrusions est une liaison de transfert d'énergie transcutané, TET,
dans lequel, en particulier, au moins l'un des éléments suivants s'applique :
- ladite liaison de transfert d'énergie transcutané, TET, comprend un couplage inductif,
- ladite liaison de transfert d'énergie transcutané, TET, utilise un dispositif externe pour transférer de l'énergie audit dispositif médical implanté,
- ladite liaison de transfert d'énergie transcutané, TET, forme un canal à très courte portée,
- ladite liaison de transfert d'énergie transcutané, TET, perd plus de 25 % de sa puissance sur 10 cm et/ou perd plus de 50 % de sa puissance sur 50 cm et/ou perd plus de 90 % de sa puissance sur deux mètres.

13. Procédé selon l'une quelconque des revendications précédentes, dans lequel au moins l'un des éléments suivants s'applique :
- ledit emplacement détecté se trouve à proximité immédiate d'un dispositif externe destiné à transférer de l'énergie audit dispositif médical implanté via une liaison formant ledit premier canal de communication résistant aux intrusions,
- ledit emplacement détecté se trouve à moins de 10 cm d'un dispositif externe destiné à transférer de l'énergie audit dispositif médical implanté via une liaison formant ledit premier canal de communication résistant aux intrusions.

14. Procédé selon l'une quelconque des revendications précédentes, dans lequel au moins l'un des éléments suivants s'applique :
- un dispositif externe destiné à transférer de l'énergie audit dispositif médical implanté via une liaison formant ledit premier canal de communication résistant aux intrusions est amené à proximité dudit emplacement détecté ; et
- une liaison de communication entre ledit dispositif médical implanté et ledit dispositif externe s'ouvre automatiquement en raison de la proximité dudit dispositif médical implanté par rapport audit dispositif externe.

15. Dispositif implantable configuré pour mettre en œuvre le procédé selon l'une quelconque des revendications 1 à 14 et configuré pour réaliser une ou plusieurs parmi une stimulation cardiaque, une défibrillation cardiaque et l'application au cœur de signaux de modulation de la contractilité cardiaque.
